# EUROPEAN PATENT SPECIFICATION

(11) **EP 2 930 246 B1**
(45) Date of publication and mention of the grant of the patent: **14.11.2018**
(21) Application number: 14163779.3
(22) Date of filing: 07.04.2014
(51) Int. Cl.: C12Q 1/68

(54) **Novel single nucleotide polymorphisms associated with prostate cancer**
Neue mit Prostatakrebs assoziierte Einzelnukleotidpolymorphismen
Nouveaux polymorphismes nucléotidiques simples associés au cancer de la prostate

(43) Date of publication of application: 14.10.2015
(73) Proprietor: QIAGEN GmbH, 40724 Hilden (DE); KTB-Tumorforschungs GmbH, 79106 Freiburg (DE)
(72) Inventor: Massing, Ulrich, 79249 Merzhausen (DE); Küllenberg, Daniela, 79289 Horben (DE); Wieczorek, Georg, 40699 Erkrath (DE); Tischler, Patrick, 40477 Düsseldorf (DE); Bahr, Andre, 42655 Solingen (DE)
(74) Representative: Uexküll & Stolberg

(56) References cited:
- Snpdev: "Reference SNP (refSNP) Cluster Report: rs2678379", , 25 September 2001 (2001-09-25), XP055131188, Retrieved from the Internet: URL:http://www.ncbi.nlm.nih.gov/projects/S NP/snp_ref.cgi?rs=2678379 [retrieved on 2014-07-23]
- FRADET V ET AL: "DIETARY LONG-CHAIN OMEGA-3 FATTY ACIDS, COX2 GENE POLYMORPHISM, AND AGGRESSIVE PROSTATE CANCER", JOURNAL OF UROLOGY, LIPPINCOTT WILLIAMS & WILKINS, BALTIMORE, MD, US, vol. 179, no. 4, 1 April 2008 (2008-04-01) , page 149, XP025797110, ISSN: 0022-5347, DOI: 10.1016/S0022-5347(08)60427-9 [retrieved on 2008-04-01]
- MARIA HEDELIN ET AL: "Association of frequent consumption of fatty fish with prostate cancer risk is modified by COX-2 polymorphism", INTERNATIONAL JOURNAL OF CANCER, vol. 120, no. 2, 25 October 2006 (2006-10-25), pages 398-405, XP055041268, ISSN: 0020-7136, DOI: 10.1002/ijc.22319
- V. FRADET ET AL: "Dietary Omega-3 Fatty Acids, Cyclooxygenase-2 Genetic Variation, and Aggressive Prostate Cancer Risk", CLINICAL CANCER RESEARCH, vol. 15, no. 7, 1 January 2009 (2009-01-01), pages 2559-2566, XP055041370, ISSN: 1078-0432, DOI: 10.1158/1078-0432.CCR-08-2503
- Katarina Augustsson ET AL: "2003;12:64-67. Cancer Epidemiol Biomarkers Prev A Prospective Study of Intake of Fish and Marine Fatty Acids", American Association for Cancer Research. cebp.aacrjournals.org Downloaded from on July, 1 January 2003 (2003-01-01), XP055131426, Retrieved from the Internet: URL:http://cebp.aacrjournals.org/content/1 2/1/64.full.pdf#page=1&view=FitH [retrieved on 2014-07-24]

### SEQUENCE LISTING

<110> Qiagen GmbH KTB Tumorforschungs GmbH
<120> Novel single nucleotide polymorphisms associated with cancer
<130> 93924
<160> 8
<170> PatentIn version 3.5
<210> 1
   <211> 201
   <212> DNA
   <213> Homo sapiens
<400> 1
   cacttaaaat taaagccttc ctaggactga aaactggaag aggagtgggg ggaagagaaa      60
   gaagaggaga gagaagaaga gtcaggaaat gacagatagg gaaagaagaa atagcaaaaa     120
   aggcaagttg gagcccaccc tgcagagacc cagaatccca acatcagtac gcattctcca     180
   agcccatgac aaacttttag t                                               201
<210> 2
   <211> 201
   <212> DNA
   <213> Homo sapiens
<400> 2
   tctgaagcca tgagctcatt gcctacaaaa tgacaggaga tttttaaggt aatgggcttg 60
   gatgagcctc aaagagcaat gaacattagg caaaaatacc gatttgacaa gttaattatt 120
   aagctggaca atgcactgaa agttaaaaat aaataacaga aaattatgaa tcttcgttgc 180
   cagtcactga tcactgtcca t 201
<210> 3
   <211> 201
   <212> DNA
   <213> Homo sapiens
<400> 3
   atctaatgtg aaaagaggag attggatttt cagaatactg tatagcttgc caaaagtagg 60
   tacttcaatt gtgtgtgaga tgtggggaag ctggaattct ggtatgtgaa ggtcaggaac 120
   ttgaaaatca ttaaggttga gagttgggat tatgaattct ggaattgcga tttctggtaa 180
   acggaagtct ggcagggtga t 201
<210> 4
   <211> 201
   <212> DNA
   <213> Homo sapiens
<400> 4
   agaagcttcc tgaagctcgt tttgaataaa ttcattgaac ttatgaagat taaggcatag 60
   gttttctttc aacaatttaa aaacatatgg gatataatca ttgaagattg tgttgatctc 120
   atcttggata taattaataa gataagtaaa tttcatctct ttcagctgtt taatgttatc 180
   ttctattagt tggaaaatga a 201
<210> 5
   <211> 201
   <212> DNA
   <213> Homo sapiens
<400> 5
   actaaaagtt tgtcatgggc ttggagaatg cgtactgatg ttgggattct gggtctctgc 60
   agggtgggct ccaacttgcc ttttttgcta tttcttcttt ccctatctgt catttcctga 120
   ctcttcttct ctctcctctt ctttctcttc cccccactcc tcttccagtt ttcagtccta 180
   ggaaggcttt aattttaagt g 201
<210> 6
   <211> 201
   <212> DNA
   <213> Homo sapiens
<400> 6
   atggacagtg atcagtgact ggcaacgaag attcataatt ttctgttatt tatttttaac        60
   tttcagtgca ttgtccagct taataattaa cttgtcaaat cggtattttt gcctaatgtt       120
   cattgctctt tgaggctcat ccaagcccat taccttaaaa atctcctgtc attttgtagg       180
   caatgagctc atggcttcag a                                                 201
<210> 7
   <211> 201
   <212> DNA
   <213> Homo sapiens
<400> 7
   atcaccctgc cagacttccg tttaccagaa atcgcaattc cagaattcat aatcccaact        60
   ctcaacctta atgattttca agttcctgac cttcacatac cagaattcca gcttccccac       120
   atctcacaca caattgaagt acctactttt ggcaagctat acagtattct gaaaatccaa       180
   tctcctcttt tcacattaga t                                                 201
<210> 8
   <211> 201
   <212> DNA
   <213> Homo sapiens
<400> 8
   ttcattttcc aactaataga agataacatt aaacagctga aagagatgaa atttacttat        60
   cttattaatt atatccaaga tgagatcaac acaatcttca atgattatat cccatatgtt       120
   tttaaattgt tgaaagaaaa cctatgcctt aatcttcata agttcaatga atttattcaa       180
   aacgagcttc aggaagcttc t                                                 201

## Description

The present invention relates to a method for identifying an individual who benefits from a high omega-3-fatty acid (n-3-FA) diet when being afflicted with prostate cancer. The method comprises the detection of at least one specific nucleotide at a single nucleotide polymorphism position in a nucleic acid sample obtained from an individual. The presence of one or more of the nucleotides at the corresponding single nucleotide polymorphism positions in the sample indicates that the respective individual benefits from an n-3-FA diet when being afflicted with prostate cancer. The present invention also provides methods for determining the responsiveness of a prostate cancer disease to a high n-3-FA diet and for identifying individuals having a reduced risk of developing an aggressive cancer type and/or metastasis when being subjected to a high n-3-FA diet.

### BACKGROUND OF THE INVENTION

Dietary factors have been found to play an important role during a number of different diseases, such as cancer. In particular, dietary fats attracted considerable attention in research during the past years. For example, an important study of Augustsson and co-workers showed that a high fish intake of more than 3 times a week led to a reduction of metastatic prostate cancer, while it did not influence the overall prostate cancer incidence [1]. This effect was confirmed in a meta-analysis, in which high fish consumption was found to lower the incidence of metastatic prostate cancer by 44% and its mortality by 63% [2]. The anti-metastatic effect has been shown to be mainly attributable to the omega-3-fatty acids (n-3-FAs) found in fish [3].

The mechanisms by which n-3 FAs may impede the formation of metastases are probably based on the fact that these fatty acids (FAs) serve as precursors in the biosynthesis of eicosanoids. Eicosanoids are tissue hormones with autocrine or paracrine actions which are involved in many physiological processes such as platelet function, immune responses and pain modulation. The dysregulation of eicosanoid biosynthesis has been linked to several pathological conditions, such as inflammation, infertility, allergy, degenerative diseases, atherosclerosis, ischemia, metabolic syndrome and, most importantly, cancer [4].

During eicosanoid biosynthesis, FAs have to be liberated from membrane phospholipids by lipolytic enzymes, which are predominantly phospholipases A2. Thereafter, cyclooxygenases (COX) or lipooxygenases (LOX), which are the first step enzymes in the transformation of n-3 or n-6 FAs, convert the previously liberated FAs into different eicosanoids. It has been found that eicosanoids derived from n-3 FAs are considerably less pro-metastatic and less inflammatory when compared to eicosanoids derived from n-6 FAs [5].

An important example for the different effects which can be exerted by eicosanoids derived from n-3 FAs or n-6 FAs, respectively, is prostaglandin E (PGE). While PGE₂ which is derived from the n-6 FA arachidonic acid (AA) is highly active in promoting metastatic spread and tumor growth, its counterpart PGE₃ which is derived from the n-3 FA eicosapentaenoic acid (EPA) is less active. This and the fact that the conversion of EPA is faster than the conversion of AA by the same enzyme (COX) results in an effective anti-metastatic effect of n-3 FAs. Thus, the n-3/n-6 FAs ratio in the cellular membranes defines whether an increased eicosanoid biosynthesis, as shown for many aggressively growing cancer cells, results in a pro- or rather anti-metastatic effect.

It has, however, been demonstrated in the Augustsson study that not all cancer patients appear to benefit from a high n-3-FA diet. While the incidence of metastatic prostate cancer and its mortality is considerably reduced by n-3-FA consumption in about 50% of the prostate cancer patients (referred to as "omega-sensitive"), no improvement was observed in the remaining patient group. Similar observations were also made in other studies. For example, it has been shown that the response rate in first-line chemotherapy of patients with advanced small cell lung cancer can be increased in about half of the study population if these patients were supplemented with fish oil [12]. Accordingly, it appears that omega-sensitivity is restricted to only about one half of a given population.

It would be highly desirable to provide a method which allows for a simple and reliable identification of omega-sensitive individuals. The identification of such individuals, even before they have been diagnosed with a disease like cancer, would provide the advantage that therapies can be tailored more individually once the disease has occurred. For example, if a freshly diagnosed prostate cancer patient with a low-medium Gleason-Score is identified as omega-sensitive, it may be advisable to abstain from classical therapies like radiation or surgery but instead subject the patient to a diet which is high in n-3-FA and low in n-6-FA and monitoring the status of the disease in regular intervals (active surveillance). Accordingly, knowledge regarding the omega-sensitivity would be an important tool for deciding between alternative therapy options and could improve current therapies in terms of efficacy and reduced side effects [12].

Fradet et al. (2008), Journal of Urology, vol. 179, no. 4, page 149; Hedelin et al. (2006), International Journal of Cancer, vol. 120, no. 2, pages 398-405; and Fradet et al. (2009), Clinical Cancer Research, vol. 15, no. 7, pages 2559-2566 describe a method for identifying prostate cancer patients who benefit from a high omega 3 fatty acid diet by detecting a single nucleotide polymorphism (SNP) in the COX2 gene in the genome of said patients.

It was therefore an objective of the present invention to provide means and methods for the identification of "omega-sensitive" individuals, i.e. individuals who benefit above-average from an n-3-FA diet when being afflicted with prostate cancer. In the course of the invention, it has been surprisingly found that certain single nucleotide polymorphisms (SNPs) in the ApoB100 gene are independently associated with omega-sensitivity of the respective individual. Thus, the detection of one or more of these SNPs can be used for determining whether a given individual is omega-sensitive or not. Individuals with one or more of these SNPs will have an improved benefit from intake of n-3-FA and can qualify for active surveillance as opposed to immediate aggressive therapy.

### BRIEF DESCRIPTION OF THE DRAWINGS

Figure 1 shows the initial median values of lipids before supplementation with marine phospholipids (MPL). The last figure shows a statistically significant difference (p<0.01) in LDL initial values between subjects taking a lipid lowering medication (n=34) and those without medication (n=90).
Figure 2 shows the change in total cholesterol and LDL in the different groups of subjects. Upper figure: Median relative change of total cholesterol after 3 months of MPL supplementation in each group of subjects. The results show a statistically significant difference (p<0.05) between patients with metastasized PCa (n=18) and patients with PCa in remission/active surveillance (n=45). Lower panel: Median relative change of LDL cholesterol after 3 months of MPL supplementation in subjects with active PCa (n=38) and inactive PCa (n=76).
Figure 3 shows the results of the fatty acid analysis. The median relative values of each FA before and after MPL supplementation in the study population are shown.
Figure 4 shows the incidence of variations in the 4 SNP positions of APOB 100 in the different groups of subjects.
Figure 5 shows the differences of MPL supplementation on lipoproteins between subjects with and without the T/T-G/G-G/G-G/G allele variation.

### DETAILLED DESCRIPTION

The invention is defined by the appended claims. In the course of the present study it was found that MPL supplementation in cancer patients as well as men without cancer led to significant differences in the levels of low-density lipoprotein (LDL) and lysophosphatidylcholine (LPC) depending on the state of the cancer disease. Patients with metastasized or active prostate cancer did not show any LDL or LPC accumulation. In contrast, a clear accumulation of LDL and LPC was observed in healthy individuals or in patients having an inactive cancer form (see Example 2 below). It can be assumed that growing or metastasizing tumor cells are characterized by a higher lipoprotein metabolism compared to inactive or healthy cells. There are indications in the scientific literature that LDL plays a role in the development of prostate cancer, possibly initiated by over-expression of LDL receptors in tumor cells. Such over-expression would satisfy the cells' increased lipid demand for proliferation.

The putative higher LPC uptake by metastatic tumor cells observed by the inventors are also in line with earlier studies showing that metastatic tumor cells rapidly catabolize LPC and incorporate the resulting free FA into their cellular membranes, thereby changing its membrane FA composition according to the FA composition of the LPC species [6]. Also, it was demonstrated in clinical trials that LPC levels were decreased in patients with colorectal cancer [7]. Thus, the obviously high LPC demand of aggressively growing tumor cells would explain the missing LPC increase observed in the group of patients with metastasized prostate cancer.

Based on the results presented in the below examples, a hypothesis can be proposed which explains the prevention of metastatic tumor growth in more than 40% of prostate cancer patients that consumed fish. It may be possible that aggressively growing prostate cancer cells develop the ability to take up high amounts of LDL and that during metastatic transformation those cells develop the additional property to take up and metabolize high amounts of LPC. Thus, LDL and LPC in plasma supply metastatic cancer cells with the FAs required for growth. Due to the higher eicosanoid synthesis of actively growing tumor cells the n-3/n-6 ratio is the decisive factor for the development of metastasis in prostate cancer. Thus, depending on which kind of FAs are predominantly contained in the diet (n-6 FAs vs. n-3 FAs), the "quality" (n-3/n-6 FA ratio) and "quantity" of the LDL and LPC particles can be modulated. Since metastatic tumor cells seem to have a higher LDL and LPC uptake, the diet strongly determines the n-3/n-6 FA ratio in metastatic tumor cells and thereby influences if cells either synthesize pro- or anti-metastatic eicosanoids. LDL and LPC could thus be regarded as Trojan horses, which become dangerous to the metastasizing tumor cells if they were enriched with n-3-FA.

The inventors were able to correlate the increases in LDL, LPC and total cholesterol in response to an n-3 FAs diet to certain genetic variations in the ApoB100 gene of individuals that underwent the study. ApoB 100 is the main apolipoprotein found in chylomicrons and LDL, which in turn are the major factors that contribute to the overall cholesterol level. Based on the information provided herein, it is possible to determine on the basis of a simple gene test whether or not an individual is omega-sensitive. This information is particularly relevant in individuals that suffer from a cancer disease, such as prostate cancer.

Omega sensitivity may explain that a number of diseases and indispositions have been described in the literature where the intake of n-3-FAs was found to improve or ameliorate the conditions in approximately half of the patients treated with 3-FA, whereas the other half could evidently not benefit from n-3-FAs intake. Such diseases and indispositions include, for example, chronic inflammations [14-16], Alzheimer disease [17], depression [18], attention deficit hyperactivity disorder (ADHD) [19], premenstrual syndrome [20], hyperlipidemia [21], disturbances of the lipid metabolism [21], metabolic syndrome or diabetes [22-23], cardiovascular diseases [24], addiction [25], and overweight [26].

Based on the SNPs identified by the present invention, it is feasible to identify individuals who respond to a high n-3-FA-diet with an increased production of LDL particles and LPC that carry n-3-FA. These individuals particularly benefit from the n-3-FA-diet when afflicted with prostate cancer.

In a first aspect, the present invention thus relates to a method for identifying an omega-sensitive individual, i.e. an individual who benefits from a high n-3-FA diet when being afflicted with prostate cancer The method comprises the following steps:
(a) providing a nucleic acid sample of the individual to be tested;
(b) detecting the presence of at least one of the following single nucleotide polymorphisms (SNPs): G/G in position 101 of SEQ ID NO:1, G/G in position 101 of SEQ ID NO:2, G/G in position 101 of SEQ ID NO:3, or T/T in position 101 of SEQ ID NO:4, or at least one of the complements thereof, at the corresponding SNP position(s) in the nucleic acid sample,
wherein the presence of at least one of the SNPs at the corresponding SNP position(s) indicates that said individual benefits from a high n-3-FA diet when being afflicted with prostate cancer.

The method can be carried out with an individual who has been positively diagnosed with prostate cancer. For such an individual, the determination of the omega-sensitivity status is useful, as it provides information that can be used for selecting a particular treatment regimen for treating the disease or disposition. For example, where a cancer patient is positive for one or more of the SNPs depicted in any of SEQ ID NO: 1-4, this means that the course of the disease can be positively influenced by a high n-3-FA diet in said patient, because the patient belongs to a subgroup of patients who benefits from high n-3-FA intake. At an early stage of the disease, it may be decided then not to subject the patient to an aggressive therapy like radiation, chemotherapy or surgery. Instead, it might be sufficient to put this patient to a high n-3-FA diet and closely monitor the disease in this patient. In addition, these patients should preferably also reduce the uptake of pro-metastatic n-6-FA. On the other hand, where a cancer patient does not contain any of the SNPs identified herein, it must be concluded that a high n-3-FA diet is not very effective in controlling the disease in said patient. Such a patient should be treated according to the standard guidelines for the respective cancer type, e.g. by means of radiation, chemotherapy or surgery.

The method of the invention is also useful for being applied to a healthy individual, i.e. an individual who does not suffer from a prostate cancer disease. In healthy individuals, the determination of the omega-sensitivity status can be performed for precautionary purposes. If the tested individual contracts a disease at a later stage of his or her life, it will be advantageous if information regarding the omega-sensitivity status is available to the practitioner for deciding whether a dietary treatment approach is promising. Furthermore, if an individual turn out to be omega-sensitive, a change in diet toward higher doses of n-3-FA or the consumption of n-3-FA-supplements might result in primary prophylaxis against prostate cancer.

Several studies conducted in prostate cancer patients revealed that omega-sensitive patients were associated with a considerably lower risk for disease progression when being subjected to a high n-3-FA diet. In particular, the risk of developing metastasis was significantly reduced in n-3-FA-taking patients compared to patients that were not omega-sensitive [1-3] (secondary prophylaxis). The present invention for the first time allows the identification of omega-sensitive patients based on the genomic sequence of their ApoB100 gene.

Accordingly, in a further aspect the present invention relates to a method for identifying an individual who has a reduced risk of developing an aggressive prostate cancer type and/or metastasis when being afflicted with a prostate cancer disease and subjected to a high omega-3-fatty acid (n-3-FA) diet. This method comprises the following steps:
(a) providing a nucleic acid sample of said individual;
(b) detecting the presence of at least one of the following single nucleotide polymorphisms (SNPs): G/G in position 101 of SEQ ID NO:1, G/G in position 101 of SEQ ID NO:2, G/G in position 101 of SEQ ID NO:3, or T/T in position 101 of SEQ ID NO:4, or at least one of the complements thereof, at the corresponding SNP position(s) in the nucleic acid sample,
wherein the presence of at least one of the SNPs at the corresponding SNP position(s) indicates that said individual has a reduced risk of developing an aggressive prostate cancer type and/or metastasis.

The information resulting from the new test of the invention can also be directly used to the benefit of a prostate cancer patient for ameliorating the symptoms, such as and tumor-related cachexia and fatigue, and reducing the severity of a prostate cancer disease. Specifically, if a prostate cancer patient is positively tested for the presence of one or more of the SNPs identified by the present invention, the diet of this patient should immediately be adapted to be high in n-3-FA and low in n-6-FA. The change in the diet will effectively ameliorate or even suppress the progression of the disease and the formation of metastasis.

In a still further aspect the invention relates to a method of assigning a patient to a diet suitable for reducing the severity of prostate cancer, comprising
(a) providing a nucleic acid sample of a patient who has been diagnosed with prostate cancer;
(b) detecting the presence of at least one of the following single nucleotide polymorphisms (SNPs): G/G in position 101 of SEQ ID NO:1, G/G in position 101 of SEQ ID NO:2, G/G in position 101 of SEQ ID NO:3, or T/T in position 101 of SEQ ID NO:4, or at least one of the complements thereof, at the corresponding SNP position(s) in the nucleic acid sample,
(c) assigning said patient to a high omega-3-fatty acid (n-3-FA) diet if at least one of the nucleotides at the corresponding SNP position(s) is detected in step (b).

In a still further aspect, the invention relates to a method of determining the responsiveness of a cancer disease to a high omega-3-fatty acid (n-3-FA) diet, comprising
(a) providing a nucleic acid sample of a patient who has been diagnosed with a cancer disease;
(b) detecting the presence of at least one of the following single nucleotide polymorphisms (SNPs): G/G in position 101 of SEQ ID NO:1, G/G in position 101 of SEQ ID NO:2, G/G in position 101 of SEQ ID NO:3, or T/T in position 101 of SEQ ID NO:4, or at least one of the complements thereof, at the corresponding SNP position(s) in the nucleic acid sample,
(c) classifying said prostate cancer as responsive to a high omega-3-fatty acid (n-3-FA) diet if at least one of the SNPs at the corresponding SNP position(s) is detected in step (b).

In a still further aspect, the invention relates to a method for identifying an individual who is able to increase the plasma levels of lysophosphatidylcholine (LPC) and/or low-density lipoprotein (LDL) and/or total cholesterol in response to omega-3-fatty acid (n-3-FA) consumption, said method comprising
(a) providing a nucleic acid sample of said individual;
(b) detecting the presence of at least one of the following single nucleotide polymorphisms (SNPs): G/G in position 101 of SEQ ID NO:1, G/G in position 101 of SEQ ID NO:2, G/G in position 101 of SEQ ID NO:3, or T/T in position 101 of SEQ ID NO:4, or at least one of the complements thereof, at the corresponding SNP position(s) in the nucleic acid sample,
wherein the presence of at least one of the SNPs at the corresponding SNP positions(s) indicates that said individual is able to increase the plasma levels of lysophosphatidylcholine (LPC) and/or low-density lipoprotein (LDL) and/or total cholesterol in response to omega-3-fatty acid (n-3-FA) consumption.

According to the invention, the individual to be tested by one of the above methods of the invention will preferably be a human. However, the methods are not restricted to humans. Instead, it will likewise be possible to use these methods with all kinds of primates, such as lemurs, monkeys, orang-utans, gorillas, bonobos, and chimpanzees. The skilled person will be readily able to identify the gene loci provided herein for humans in other primates.

In the context of the invention, the term "high n-3-FA diet" refers to a diet which, either by itself or by way of supplementation, is rich in n-3 FA. A natural diet which contains high amounts of n-3 FA is fish, in particular salmon, herring, tuna and mackerel. The consumption of fish 3-4 times a week is therefore effective, e.g. in reducing the risk of developing an aggressive cancer type and/or metastasis in an omega-sensitive patient afflicted with a cancer disease. It is also possible to ingest high amounts of n-3 FA in the form of fish oil. In practice, however, it turned out that a high fish intake or the intake of fish oil is difficult, since a strict diet is not easily followed by all patients. For this reason, a high n-3-FA diet may also be achieved by relying on n-3 FA supplementation. Supplementation includes both the admixture of n-3-FA into common food items and the separate administration of n-3-FA, e.g. in the form of capsules. For supplementation, the n-3 FA can be used in all forms which allow the admixture into food items or their direct administration. For example, the n-3 FA can be used in the form of n-3 FA methyl esters or as free n-3 FA.

In a particularly preferred embodiment of the invention, the n-3-FA is administered in the form of marine phospholipids (MPL). MPL are a type of glycerophospholipids (GPLs) containing long chain n-3 FAs bound to phospholipids (PLs). Studies have demonstrated beneficial health effects, since the uptake and metabolism of n-3 FAs bound to PLs turned out to be more efficient than n-3 FAs bound to triglycerides [13]. As such, MPL have proven to be a good fish substitute. In addition, MPL have been proven to be much better tolerated than fish oil or n-3 FA methyl esters. In an even more preferred embodiment, MPL are administered in the form of soft gelatine capsules. Such capsules can be obtained from different manufacturers, such as Membramed Health Food GmbH (Hamburg, Germany).

As used herein, a high n-3-FA diet preferably means that the overall weekly intake of n-3 FAs will be in the range of 0.7 to 35 g. Such intake can be achieved by consuming fish 3-4 times a week. If the n-3 FAs are to be delivered by food supplements, on a daily basis, the overall daily intake of n-3 FAs will be in the range of 0.1 and 5 g n-3 FAs, preferably between 0.5 and 5 g, more preferably between 1.0 and 3 g, such as 1 g or 2 g. In another preferred embodiment, the overall daily intake of n-3 FAs will be in the range of 0.4 and 0.5 g n-3 FAs. When food supplements are separately administered, e.g. in the form of capsules, the overall daily intake can be achieved by several administrations per day. For example, to achieve an overall daily intake of 1.5 g n-3 FAs, three capsules containing 500 mg n-3 FAs can be administered in the course of the day.

The above methods are essentially based on the insight that the SNPs shown at nucleotide position 101 in SEQ ID NO:1-4 are associated with the individual's ability to benefit from a high n-3-FA diet when being afflicted with certain diseases or indispositions, in particular cancer, more particular prostate cancer. As used in the context of the present invention, a single nucleotide polymorphism (SNP) refers to a single nucleotide position in a nucleic acid, such as a DNA or RNA, which is occupied by alternative nucleotides in different alleles in a given population. The position of the SNP is usually flanked by highly conserved sequences of the allele. A SNP can be located in a coding region of the DNA of the individual, in a non-coding region or in an intergenic region. Where the SNP is located in the coding region, it may be synonymous or non-synonymous. A synonymous or silent SNP is one that due to the degeneracy of the genetic code does not result in an amino acid change compared to the reference allele. In contrast, a non-synonymous nucleotide substitution leads to an amino acid replacement which may give rise to an altered expression product. Alternatively, it may also lead to a premature termination of the translation process if a stop codon is formed by the substitution at the polymorphic site. It is also possible that the substitution destroys a naturally occurring stop codon at the polymorphic site which leads to an extended read-through expression product. In contrast, where a SNP is not in a coding region of the genome, it may nevertheless affect protein expression, for example by influencing RNA splicing or DNA binding to transcription factors, and the like.

A biallelic individual, such as a human, has two alleles for each gene and therefore may be homozygous or heterozygous with respect to a given allele at a particular SNP position. In a biallelic organism, such as a human, a single nucleotide polymorphism can result in three different genotypes. For example, an A/C polymorphism can give rise to a first homozygous genotype "AA", a second homozygous genotype "CC" and a heterozygous genotype "AC".

Each of the above methods of the invention comprises a detecting step in which the presence of at least one of the nucleotides shown in position 101 of SEQ ID NO: 1-4 are detected at the corresponding SNP positions in the nucleic acid of the individual to be tested. Alternatively, where the detection is performed on the basis of the complementary DNA strand, the methods of the invention will include the detection of at least one nucleotide which is complementary to the nucleotide shown in position 101 of SEQ ID NO: 1-4. If the detection is performed based on the complementary DNA strand, the detection will be directed to nucleotides shown in position 101 of SEQ ID NO:5-8.

The polynucleotides shown in SEQ ID NOs: 1-4 each represent a section of human genomic DNA containing an SNP that was found to be associated with omega-sensitivity. The polynucleotides shown in SEQ ID NOs:5-8 represent the reverse complementary sequences of SEQ ID NOs: 1-4, respectively. The polymorphic site is located at nucleotide position 101 in each of SEQ ID NO: 1-8, while the flanking sequences upstream (i.e. nucleotides 1-100) and downstream (i.e. nucleotides 102-201) of the SNP position represent human genomic DNA which is normally conserved between different individuals. These flanking sequences are depicted herein to allow for the location of the SNPs within the genomic sequence of the individual to be tested and the generation of primers and probes useful in detection of the SNP.

For each of the SNPs identified herein in the context with omega-sensitivity, there are two possible allele forms, which are referred to herein as the "omega allele" and the "non-omega allele. The omega allele is the allele which is found in individuals who respond to a high n-3-FA-diet by increasing plasma levels of LDL and/or total cholesterol. In particular, the omega allele is found in cancer patients that have been shown to benefit from a high n-3-FA diet. In contrast, the non-omega allele was found to be present in individuals who do not respond to a high n-3-FA-diet by increasing plasma levels of LDL and/or total cholesterol. In particular, the non-omega allele is found in cancer patients who are not affected by a high n-3-FA diet. The sequences of SEQ ID NO: 1-4, as well as their reverse complement sequences in SEQ ID NO:5-8, represent the omega allele, i.e. the detection of these sequences indicates that the tested individual is omega-sensitive.

According to the invention, the presence of at least one of the nucleotides shown in position 101 in SEQ ID NO:1-4 or SEQ ID NO:5-8 is considered as detected, if a biallelic individual, such as a human, carries two of the omega alleles, i.e. the individual is homozygous with respect to said omega allele. If the individual is homozygous, both alleles comprise the sequence of the omega allele, whereas in a heterozygous individual only one allele corresponds to the omega allele while the other is a non-omega allele.

Accordingly, the detection of one of the SNPs by the methods described above is considered to be achieved only if both alleles found in the individual are omega alleles with respect to this particular SNP.

**Table 1: Sequences containing SNPs predictive for omega-sensitivity**

| **SEQ ID NO:** | **Chromosome** | **Position on the chromosome** | **omega Allele** | **Non-omega Allele** |
|---|---|---|---|---|
| 1 | 2 | 21226560 | G | A |
| 2 | 2 | 21237544 | G | A |
| 3 | 2 | 21231524 | G | A |
| 4 | 2 | 21225281 | T | C |
| 5 | 2 | 21226560 | C | T |
| 6 | 2 | 21237544 | C | T |
| 7 | 2 | 21231524 | C | T |
| 8 | 2 | 21225281 | A | G |

Table 1 indicates the exact position of the sequences of SEQ ID NOs:1-8 on chromosome 2. The nature of the omega allele and the non-omega allele are also provided. It can be seen in Table 1 that the sequences of SEQ ID NOs: 1-4, and their corresponding complements of SEQ ID NO:5-8, refer to different SNP locations in chromosome 2. The detection of one of the SNPs shown in SEQ ID NO: 1-4 can be achieved by identifying in the nucleic acid of the individual to be tested the nucleotide which corresponds to position 101 of any of the sequences depicted in SEQ ID NO: 1-4. Each of the sequences set forth in SEQ ID NO: 1-4 are located on the same DNA strand. As stated above, the detection may also be performed on the basis of one or more of the complement sequences provided in SEQ ID NO:5-8.

According to the invention, the above methods can include the testing of one or more of the SNPs shown in SEQ ID NO:1-4. Although the SNPs of SEQ ID NOs:1-4 provide reliable results when tested independently from each other, the significance of the results can be improved by testing a nucleic acid sample for more than one of the SNPs. According to the present invention, it is thus preferred that step (b) of the above methods includes the testing of combinations of the different SNPs provided in SEQ ID NOs: 1-4. The combination to be tested according to the present invention may comprise two, preferably three, and even more preferably all four SNP positions. For example, step (b) of the above described methods can include the detection of the SNP of SEQ ID NOs:1 in combination with the SNP of SEQ ID NOs:2, the SNP of SEQ ID NOs:1 in combination with the SNP of SEQ ID NOs:3, the SNP of SEQ ID NOs: 1 in combination with the SNP of SEQ ID NOs:4, the SNP of SEQ ID NOs:2 in combination with the SNP of SEQ ID NOs:3, the SNP of SEQ ID NOs:2 in combination with the SNP of SEQ ID NOs:4, or the SNP of SEQ ID NOs:3 in combination with the SNP of SEQ ID NOs:4. Of course, the detection at some or even all of the SNP positions can be performed by analyzing the complementary DNA strand. Accordingly, the detection may include one or more of the sequences depicted in SEQ ID NO:5-8 rather than to the corresponding sequences of SEQ ID NO:1-4.

When combinations of three SNPs are used, these combinations may include the SNP of SEQ ID NOs:1 in combination with the SNPs of SEQ ID NOs:2 and SEQ ID NOs:3, the SNP of SEQ ID NOs:2 in combination with the SNPs of SEQ ID NOs:3 and SEQ ID NOs:4, the SNP of SEQ ID NOs:1 in combination with the SNPs of SEQ ID NOs:3 and SEQ ID NOs:4, or the SNP of SEQ ID NOs:1 in combination with the SNPs of SEQ ID NOs:2 and SEQ ID NOs:4. According to a particularly preferred embodiment, step (b) of the above methods comprise the detection of all four SNPs set forth in SEQ ID NOs:1-4. Again, the detection at some or all of the SNP positions can be performed by analyzing the complementary DNA strand. Accordingly, the detection may be directed to one or more of the sequences depicted in SEQ ID NO:5-8 rather than to the sequences of SEQ ID NO:1-4.

It was found that the four SNPs identified herein are often linked with each other in the sense that individuals comprise all four omega alleles at the same time. Also for this reason, the detection of a single SNP is highly reliable for determining whether the patient is omega-sensitive or not.

The methods of the present invention are performed with a nucleic acid sample obtained from the individual to be tested. The nucleic acid is preferably genomic DNA or a nucleic acid which is derived from genomic DNA, such as cDNA. In an alternative embodiment of the invention, the nucleic acid used for detecting the SNPs is RNA. The DNA or RNA which is subjected to SNP detection can be obtained from a cell or tissue sample of the individual to be tested, such as from biopsy material, skin cells or body fluids. In a preferred embodiment of the invention, the nucleic acid sample is derived from a blood sample of the individual to be tested. In another preferred embodiment, the nucleic acid sample is derived from a mucosa swab.

Several methods for isolating genomic DNA from eukaryotic cells or tissues have been described in the art. The genomic DNA may for example be isolated as described in standard textbooks [8]. More conveniently, commercially available kits can be used for isolating genomic DNA, for example, the QIAamp DNA mini kit (Qiagen) or the ChargeSwitch Genomic DNA Purification Kit (Invitrogen). Methods for isolating DNA usually involve the lysis of the cells of the cell or tissue sample obtained from the test person. Cell lysis may be performed by disrupting the plasma membrane of the cells, for example, by boiling the sample or by use of detergents such as SDS or the like. The RNA of the sample is then normally digested by using RNAse. Protein contaminants can be readily degraded by incubating the sample with proteinase K. Genomic DNA can be recovered by alcohol precipitation or by binding to an anion exchange column.

Alternatively, where the polymorphic site is located in a coding region of the genome, it will also be possible to detect the SNP at the RNA level. In this case, mRNA may be provided from a cell or tissue sample of the individual. Typically, the complete RNA is isolated from the cell or tissue sample. Methods for isolating RNA are well known in the art and are discussed in detail in the literature. These methods regularly involve the lysis of the cells of the respective cell or tissue samples. Cell lysis may be performed by use of detergents which are capable of disrupting the plasma membrane of the cells. For example, buffers containing guanidine thiocyanate and/or SDS may be used for cell lysis. The methods may also comprise steps in which the DNA of the cells is enzymatically digested in order to obtain pure RNA without traces of DNA which might interfere with the further downstream applications. Inhibitors of enzymes which lead to the degradation of the RNA may also be added to the lysis buffer. Kits for preparing highly purified RNA are available from several manufactures, such as Qiagen and others.

The RNA isolated from the cell or tissue sample will normally comprise different types of RNA. Preferably, the RNA obtained from the tissue sample is total RNA comprising mRNA, transfer RNA (tRNA) and ribosomal RNA (rRNA). For the methods of the present invention, it is desirable to enrich the mRNA fraction with respect to fractions of other cellular RNAs. Preferably, the mRNA is separated from other RNA molecules. Methods for enriching or purifying mRNA are known in the art. For example, an affinity chromatography using oligo(dT) or poly(U) coupled to a solid matrix, such as a cellulose or Sephadex matrix can be performed, as most mRNAs contain a poly(A) tail at their 3' terminus [9]. Poly(A)+ mRNA which has bound to the affinity matrix can be eluted using 2 mM EDTA/0.1% SDS.

Once the DNA or RNA has been isolated from the cell or tissue material, the presence of one or more of the SNP sequences of SEQ ID NOs:1-4 can be detected according to methods that are well known in the art. The detection of a specific nucleotide at a SNP site in a polynucleotide is commonly referred to as genotyping of the SNP. Several methods suitable for SNP genotyping are known in the field. Each of those methods can be used for carrying out detection step (b) of the methods of the invention described above.

In a simple embodiment, PCR-based methods are used in a first step to amplify a nucleic acid sequence comprising the putative SNP site of interest. These methods may include PCR, RT-PCR, NASBA and the like. The PCR products obtained in this way can subsequently be subjected to common nucleotide sequencing techniques. Where the nucleic acid sample to be examined is DNA, especially genomic DNA, standard PCR techniques can be used. Where the nucleic acid sample is RNA, in particular mRNA, a RT-PCR approach is used.

In another embodiment of the invention, a TaqMan PCR approach is used for SNP genotyping. TaqMan PCR is a fluorophore-based real time PCR method which detects accumulation of a specific amplified product during PCR. In TaqMan PCR, a single-stranded oligonucleotide probe is added to the reaction which is complementary to a segment of 10-50 nucleotides within the DNA template and located between the two PCR primers. A fluorophore and a quencher dye are covalently attached to the 5' and 3' ends of the probe, respectively. Alternatively, the quencher dye can also be attached to an internal nucleotide, while the fluorophore is attached to the 5' or 3' end of the probe or vice versa. The close proximity between fluorophore and quencher dye attached to the probe inhibits fluorescence emission from the fluorophore when said fluorophore is selectively excited. During DNA synthesis in the PCR, the 5' exonuclease activity of the Taq polymerase cleaves the oligonucleotide probe which is hybridized to the template DNA, thereby spatially separating the fluorophore and the quencher dye. Fluorescence is detected during PCR cycling; it is directly proportional to the fluorophore released and the amount of DNA template present in the PCR. Each fluorophore-quencher pair known in the art can be used in the methods of the present invention. Examples of suitable fluorophores are FAM (6-carboxyfluorescin), TET (tetrachlorof-luorescin) or VIC. A suitable quencher dye is TAMRA (tetramethylrhodamine). The construction and labeling of the oligonucleotide to be used in a TaqMan approach have been described in great detail in the literature.

It is likewise possible to use differently labeled probes in the same TaqMan assay. For example, one TaqMan probe specific for the omega allele of the SNP is labeled with FAM and another TaqMan probe that is specific for the non-omega allele is labeled with VIC. In such a set-up, fluorescence of FAM and no fluorescence of VIC would indicate for the presence of the omega allele, whereas fluorescence of VIC and no fluorescence of FAM would be indicative for the presence of the non-omega allele. Fluorescence of both, FAM and VIC, would be indicative for a heterozygous state. The present invention also contemplates modifications of the TaqMan method, for example, assays that use molecular beacons.

A further method for detecting a SNP in a polynucleotide sequence is described, for example, in US 4,988,617. In this method, the occurrence of a specific nucleotide is determined by an oligonucleotide ligation assay using a pair of oligonucleotide probes that selectively hybridize immediately upstream and downstream of the polymorphic site. Upon hybridization, one of the terminal nucleotides of the oligonucleotides is aligned to the site of the putative polymorphism. Where the terminal nucleotide of the probe is complementary to the nucleotide at the polymorphic site, hybridization of the oligonucleotide to the polynucleotide that contains the polymorphic site will include the terminal nucleotide of said oligonucleotide. In this case, the addition of a ligase, such as a T4 ligase, will link both oligonucleotide probes to each other. In contrast, where the terminal nucleotide of the probe is not complementary to the nucleotide at the polymorphic site, no ligation of the oligonucleotide to the polynucleotide containing the polymorphic site will occur. The ligation product derived from the linkage of the two oligonucleotides can be detected in a subsequent step, for example by a PCR reaction. Several test systems are currently available which are based on oligonucleotide ligation.

Single base extension methods can also be used for the detection of single nucleotide polymorphisms. Single base extension methods are described, e.g., in US 5,846,710, US 6,004,744, US 5,888,819 and US 5,856,092. Briefly, these methods are based on the hybridization of a primer that is complementary to a target sequence such that the 3' end of the primer is adjacent to but does not span the site of the putative SNP in the target sequence. The primer comprises a sequence which is complementary to the target polynucleotide terminating at the base that is immediately adjacent 5' to the polymorphic site. The hybridization is performed in the presence of one or more labeled nucleotides complementary to base(s) that may occupy the site of potential variation. For example, for a biallelic polymorphism two differentially labeled nucleotides can be used. Preferably, the labeled nucleotides are dideoxynucleotides. Hybridization is performed under conditions permitting primer extension if a nucleotide complementary to a base occupying the site of variation in the target sequence is present. Extension incorporates a labeled nucleotide thereby generating a labeled extended primer. If multiple differentially labeled nucleotides are used with a heterozygous target, multiple differentially labeled extended primers are obtained. Extended primers are detected providing an indication of which base(s) occupy the polymorphic site in the target sequence.

Another method for genotyping involves an invader assay [10]. In the invader assay, a cleavase (e.g., the flap endonuclease FEN) is combined with two specific oligonucleotide probes that together with the target DNA can form a tripartite structure recognized by the cleavase. The first probe, called the invader oligonucleotide is complementary to the 3' end of the target DNA. The last base of the invader oligonucleotide is a non-matching base that overlaps the SNP nucleotide in the target DNA. The second probe is an allele-specific probe which is complementary to the 5' end of the target DNA, but also extends past the 3' side of the SNP nucleotide. The allele-specific probe will contain a base complementary to the SNP nucleotide. If the target DNA contains the desired allele, the invader and allele-specific probes will bind to the target DNA forming the tripartite structure. This structure is recognized by cleavase, which will cleave and release the 3' end of the allele-specific probe. If the SNP nucleotide in the target DNA is not complementary to the allele-specific probe the correct tripartite structure is not formed and no cleavage occurs. The invader assay can be coupled with a fluorescence resonance energy transfer (FRET) system to detect the cleavage. In this case, a quencher molecule is attached to the 3' end and a fluorophore is attached to the 5' end of the allele-specific probe. If cleavage occurs, the fluorophore will be separated from the quencher molecule generating a detectable signal.

According to another embodiment the genotyping of the SNPs is carried out by a dynamic allele-specific hybridization approach [11]. This approach is based on differences in the DNA melting temperature that results from instability of mismatched base pairs. In the first step of, a genomic fragment comprising the SNP is amplified and attached to a bead through a PCR reaction with a biotinylated primer. In the second step, the amplified product is attached to a streptavidin column and washed to remove the nucleic acids which are not biotinylated. An allele specific oligonucleotide is then added in the presence of a molecule that emits fluorescence when bound to doublestranded DNA. The fluorescence intensity is measured at increasing temperature until the melting temperature (Tm) is reached. A probe that is specific for the allele of the SNP will exhibit a Tm which differs from that of an unspecific probe which allows the detection of the nucleotide at the site of polymorphism.

In another preferred embodiment of the invention, a microarray is used for detecting the presence of the SNPs shown in SEQ ID NO:1-4. A microarray refers to the orderly arrangement of spatially resolved probes, for example nucleic acid probes, on a substrate. The substrate is preferably a solid substrate having a surface with a plurality of probes attached in different known locations (spots). The spots on a microarray are normally either printed on the microarrays or synthesized by photo-lithography or by ink-jet printing. On a common microarray, there may be several thousand spots, wherein each of the spots may contain a high number of identical probes, such as nucleic acid fragments or oligonucleotides. Such microarrays typically have a density of at least 100 oligonucleotides or fragments per cm². In certain embodiments the arrays can have a density of about at least 500, at least 1000, at least 10000, at least 10⁵, at least 10⁶, at least 10⁷ oligonucleotides or fragments per cm². The support can be a glass slide or membrane on the surface of which the probes are attached at fixed locations or spots. The use of microarrays for interrogating several SNPs simultaneously has been extensively described in the art.

When using a microarray in the detection step (b) of the above methods, the nucleic acid sample to be analyzed is hybridized to the probes on the microarray. By comparing the differential extent of hybridization of the sample nucleic acid from the individual to be tested to each of the probes, it is possible to determine specific homozygous and heterozygous alleles. For example, it can be determined whether an omega allele is present or absent. Generally, the probes of the microarray are allele-specific polynucleotides. These probes can be used to distinguish nucleic acid samples of individuals carrying the non-omega allele from samples of individuals carrying the omega-allele. The probes can be arranged such that their central site is the site where the nucleotide exchanges according to the invention occur, e.g. the 8^{th} position in a probe consisting of 17 nucleotides may be specific to the omega allele but not to the non-omega allele of a SNP. Specific for the omega allele means that a nucleic acid from an individual will bind to this probe only if said individual indeed carries the omega allele.

Finally, the present disclosure also relates to kits for performing the methods of the present disclosure. In a preferred embodiment the kit comprises oligonucleotide primers which selectively hybridize to a sequence as provided in SEQ ID NOs:1-4 and allow for the amplification of a nucleic acid product which contains the SNP site of interest, i.e. nucleotide position 101. The kit may also comprise buffers, reagents and enzymes, such as polymerases or reverse tran-scriptases which are useful for detecting the SNPs described herein. Alternatively or in addition thereto, the kit may comprise suitable probes which are labeled, e.g. probes for TaqMan PCR or hybridization probes. The kit will further comprise instructions for using the components in a method for determining whether an individual is omega-sensitive.

The oligonucleotide primers contained in the kit are designed to allow specific hybridization to sequences in the vicinity of the polymorphic site, for example, in the regions upstream or downstream of nucleotide position 101 in any of the polynucleotides in SEQ ID NOs:1-4. The skilled person will be readily able to identify on the basis of the present disclosure suitable oligonucleotide primers that can be used for amplifying an SNP in one of the loci described herein. The oligonucleotides may be of any length, but will preferably be at least 10-50 nucleotides in length, for example, 10, 12, 14, 16, 18, 20, 22, 24, 26, 28, 30, 32, 34, 36, 38, 40, 42, 44, 46, 48 or 50 nucleotides in length. They can be prepared by any suitable technique known in the art. For example, they may be derived synthetically, for example by the well-known phosphoamidite method. The oligonucleotides for use in the methods of the present invention may contain modified bases, such as 5-methylcytosine, 5-hydroxymethylcytosine, 2-thiouracil, 8-thiolguanine, 7-methylguanine, 7-methyladenine, 8-azaguanine und 8-azaadenine, 7-deazaguanine, 7-deazaadenine, 3-deazaguanine and/or 3-deazaadenine. The length and composition of the oligonucleotides will depend on the particular PCR or RT-PCR method which is selected for amplifying the SNPs according to the invention. Methods for designing oligonucleotides for use as PCR or RT-PCR primers have been extensively described in the scientific literature. The PCR or RT-PCR used for SNP genotyping may be a multiplex approach.

The kit can comprise oligonucleotide primers which are useful for performing a multiplex PCR or RT-PCR. The kit is intended for use in one of the above methods, e.g. for use in a method for identifying an individual who benefits from a high omega-3-fatty acid (n-3-FA) diet when being afflicted with one of the above-mentioned diseases or indispositions, in particular a cancer disease, e.g. prostate cancer, for use in a method for identifying an individual who has a reduced risk of developing an aggressive cancer type and/or metastasis when being afflicted with a cancer disease and subjected to a high omega-3-fatty acid (n-3-FA) diet, for use in a method of reducing the severity of a cancer disease in a patient, for use in a method of determining the responsiveness of a cancer disease to a high omega-3-fatty acid (n-3-FA) diet, and for use in a method for identifying an individual who is able to increase the plasma levels of lysophosphatidylcholine (LPC) and/or low-density lipoprotein (LDL) and/or total cholesterol in response to omega-3-fatty acid (n-3-FA) consumption.

The invention will be in described in more detail by way of the following examples.

### EXAMPLES

### Example 1: Patient recruitment and MPL delivery

Between December 2009 and October 2011 a total of 159 subjects (113 patients with prostate cancer (PCa) and 46 subjects without PCa) were included into the present study. All participants gave informed written consent before study enrolment. The clinical trial was performed in Freiburg, Germany, where fish intake is usually limited. This assured a baseline level of low fish intake in the study population. Subjects were recruited at the Tumor Biology Center Freiburg, at the urological ward in the Loretto hospital Freiburg and at the urological practice U3 Freiburg.

PCa patients were included with various cancer stages, receiving different therapies, for example surgery, radiation, hormone therapy, chemotherapy or active surveillance. Inclusion criteria for PCa patients were as follows: male subjects older than 18 years with histologically proven PCa and written inform consent. Inclusion criteria for subjects without PCa were: male subjects without PCa or any other tumor, aged 70 years or older. Exclusion of PCa with any of the following criteria: negative results from a 12x punch biopsy and PSA values below 10 ng/ml; men with benign prostatic hyperplasia (BPH), in whom a transurethral resection of the prostate (TUR-P) was conducted and the histology of the recovered material was negative; or subjects with PSA levels less than 4 ng/ml and no prostate cancer family history. Exclusion criteria for all participants were: known allergy to sea-food; malabsorption; impaired coagulation or other severe internal diseases; psychiatric or CNS disorders; subjects already taking omega-3 (n-3) fatty acids (FAs) as supplement.

Participating subjects were asked to take one capsule of 500 mg Vitalipin three times a day with their meals for a period of three months. Vitalipin is a preparation of marine phospholipids (MPL) packed in soft gelatine capsules, obtained from Membramed Health Food GmbH (Hamburg, Germany). The MPL composition consists of approximately 30% phospholipids (mostly phosphatidylcholine) and other lipids with a fatty acid (FA) composition of 18% eicosapentaenoic acid (EPA) and 26% docosahexaenoic acid (DHA).

Patients and healthy subjects were examined at the beginning and at the end of the study. Examination included questions about their medical history, their present complaints, nutritional habits (especially fish intake), family history, ongoing medication, and blood sampling for routine analysis, determination of fatty acids (FAs) and LPC in plasma.

### Example 2: Analysis of blood samples

Blood samples for FAs and LPC analyses were collected as EDTA blood tubes (S-Monovette® with 1.6 mg EDTA/ml blood, Sarstedt, Germany), and 1 Serum tube (S-Monovette®, Sarstedt, Germany). One EDTA blood tube was directly stored at -80°C until SNP analysis. Two other EDTA tubes were centrifuged for 10 minutes at 2000 rpm at 4°C. The resulting plasma was stored in aliquots of 500 µl at -80°C until further analysis. The analysis of blood lipids was performed by the clinical chemistry laboratory of the Tumor Biology Center Freiburg. Blood lipids were measured at baseline and after 3 months of MPL supplementation with lipid electrophoresis. Since triglycerides and VLDL are sensible to food intake, values were not considered for analysis.

### Results:

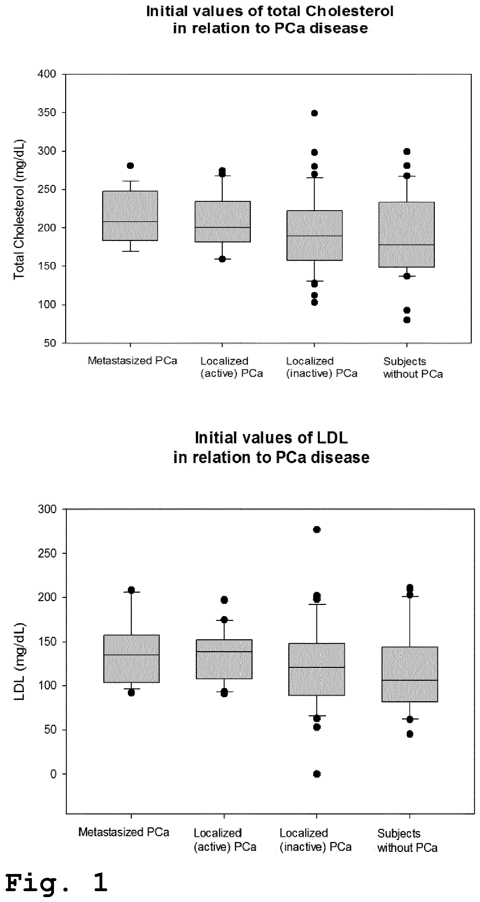

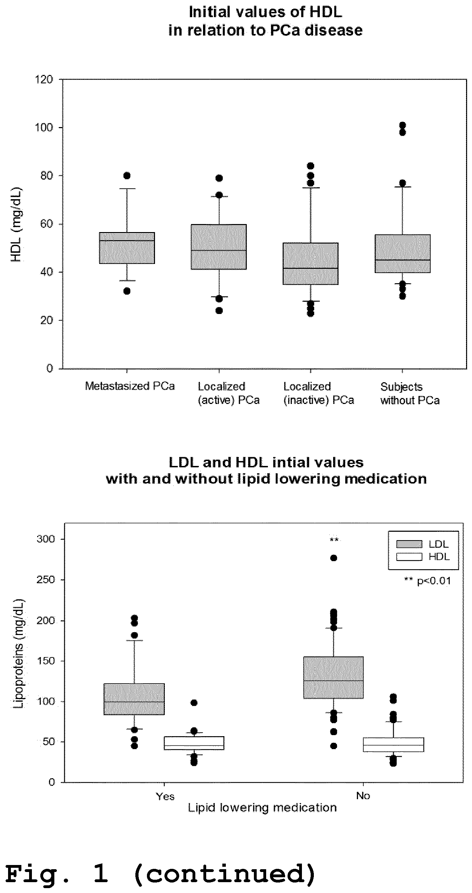

The results show that the study population had normal cholesterol levels before MPL supplementation (see Figure 1). Differences on the initial values of cholesterol were analyzed in the groups of subjects, resulting in significant differences in total cholesterol and LDL, but not in HDL levels before MPL supplementation. Subjects taking lipid lowering medication (about 38% of subjects) had significant lower LDL initial values than all other subjects, whereas HDL remained unaffected.

After MPL supplementation, the measured total cholesterol, LDL and HDL levels increased significantly in the whole study population about 15%. The results depicted in the below table are based on a lipid electrophoresis analysis with non-fasting blood samples (**significance level p<0.001).

**Table 2: Total cholesterol, LDL and HDL levels after MPL supplementation.**

| | **Before MPL (mg/dL)** | **After MPL (mg/dL)** | **Change (%)** |
|---|---|---|---|
| **Total cholesterol** Median (min-max) | 194 (80-349) | 221 (115-362) | + 14% ** |
| **LDL cholesterol** Median (min-max) | 121 (36-277) | 139 (42-282) | + 15% ** |
| **HDL cholesterol** Median (min-max) | 49 (23-106) | 57 (21-125) | + 16% ** |
| **LDL:HDL** Mean+/-SD | 2.8+/-1.3 | 2.7+/-1.4 | - |

The changes in the LDL and HDL levels were not affected if subjects were prescribed lipid lowering medication.

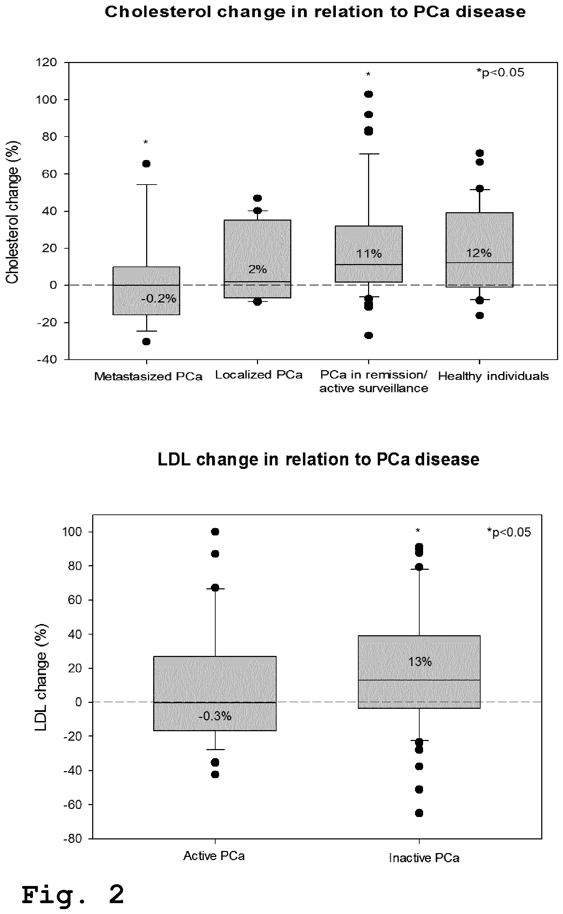

Differences in the lipid change between each group of subjects were analyzed. The results are depicted in Figure 2. The results show clearly that patients with metastasized PCa had almost no change in their total cholesterol levels after MPL supplementation, whereas all other patients and subjects without PCa had a significant increase. Detailed analysis showed that these differences were found mainly in the LDL fraction of total cholesterol. LDL levels after MPL remained almost unchanged in patients with active PCa, whereas in patients with inactive PCa, LDL increased significantly. The difference between the groups was also statistically significant (p<0.05). No significant differences were found in the HDL cholesterol change between each group of subjects, probably due to lower HDL concentration in relation to LDL blood concentration.

### Example 3: Analysis of fatty acid (FA) composition in plasma

We analyzed the FA composition in the fat fraction of blood plasma which had previously been separated into triglycerides (TGs) and phospholipids (PLs). The FA analysis of the PL fraction therefore represents a near estimation of the FAs found in lipoproteins. A total of 10 FAs in each subject were analyzed before and after MPL supplementation.

### Results:

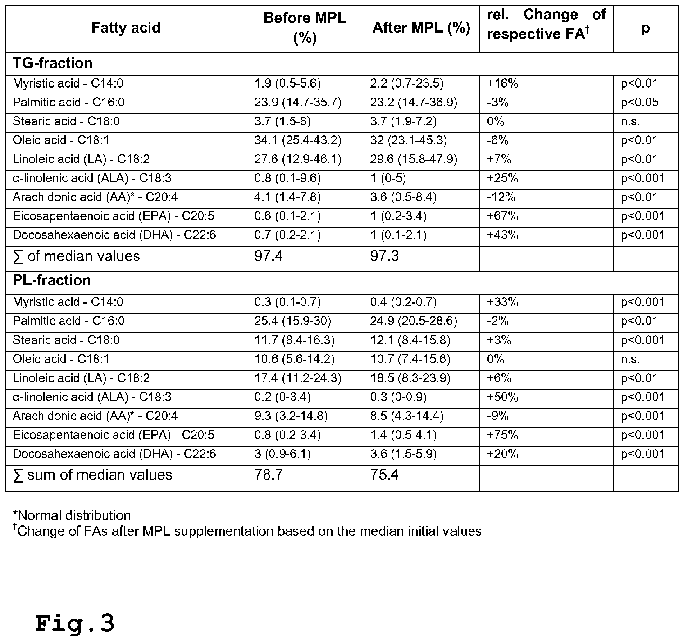

It was found that the initial values of α-linolenic acid (ALA) were significantly higher mainly in patients with metastasized PCa in contrast to all other subjects in both the TG- and PL fraction. No significant differences were observed for the initial values of all other FAs between the groups of subjects. The table in Figure 3 shows the results of the FA analysis both before and after MPL supplementation. The results show a displacement of FAs in plasma after supplementation with MPL.

From the biologically active n-3 and n-6 FAs, eicosapentaenoic acid (EPA) and docosahexaenoic acid (DHA) increased significantly after MPL supplementation in both the TG and PL fraction of plasma, whereas arachidonic acid (AA) decreased significantly. Therefore, the n-3/n-6 FA ratio improved significantly from 0.38 to 0.58, (EPA + DHA)/AA in the TG and PL fraction, with p<0.01. Linoleic acid (LA, 18:2, n-6 FA) and ALA (18:3, n-3 FA), which are precursors of the biologically active FAs EPA and AA, were shown to increase significantly (p<0.01) in both, the TG and the PL fraction of plasma, after MPL supplementation. Differences on the FA change between each group of subjects were analyzed, finding no significant differences.

### Example 4: Analysis of lysophosphatidylcholine (LPC) in plasma

Lysophosphatidylcholine (LPC) is a result of the partial hydrolysis of phosphatidylcholine (PC) after cleavage of one of the FA groups. The hydrolysis is generally achieved enzymatically by the phospholipase A2, which cleaves the FA from the 2-position of the glycerol backbone. It has been observed that tumour cells consume higher amounts of LPC to comply with their higher membrane turnover. Therefore, the effects of MPL on LPC levels in plasma were analyzed to determine if differences between the groups of subjects were present.

### Results:

Initial values of LPC were not significantly different between the groups of subjects and had a median value of 181 µM. After MPL supplementation the study population displayed a significant LPC increase from 181 µM to 223 µM (p<0.001). When observing the absolute LPC change values in the different groups of subjects, patients with metastasized PCa clearly had a LPC decrease after MPL, whereas in all other subjects LPC increased. This result was significant (p<0.05) between patients with metastasized PCa (LPC relative decrease of 7%) and all other subjects (LPC relative increase of 18%).

### Example 5: Analysis of genetic variations

A total of 12 genes of the FA metabolism, which have been shown to be involved in PCa, were analyzed in subjects enrolled into this study. These genes included the cyclooxygenase 2 (PTGS2), lipoproteinlipase (LPL), secretory phospholipase A2 (PLA2G2A), lecithin-cholesterol acyltransferase (LCAT), cytochrome P450 (CYP2J2), arachidonate 15-lipoxygenase (ALOX15), arachidonate 15-lipoxygenase type II (ALOX15B), 5-Lipooxygenase (ALOX5), 12-Lipoxygenase (ALOX12), apolipoprotein A1 (APOA1), apolipoprotein A2 (APOA2), and apolipoprotein B (APOB).

The analysis of genetic variations included SNPs as well as insertions and deletions. One EDTA blood tube was taken from each participant at study enrolment and stored at -80°C for sequence analysis. Upon analysis, DNA from leucocytes of the blood samples was isolated using the QIAamp DNA Blood Maxi Kit (QIAGEN, Hilden, Germany). The DNA was subsequently amplified by long range PCR using the LR PCR SeqTargetLongRange PCR Kit (QIAGEN, Hilden, Germany). Sample normalization was achieved by using the SeqTarget-Normalization Kit (QIAGEN, Hilden, Germany). These processing steps were performed according to the manufacturer's instructions.

After pooling the samples, "next-generation DNA sequencing" (NGS) was carried out by LGC Genomics GmbH (Berlin, Germany) using an Illumina HiSeq 2000 according to standard procedures. The concept of NGS is to first fragment the DNA into a library of small segments which are afterward sequenced simultaneously in millions of parallel reactions. The resulting strings of bases, also called reads, were quality checked, trimmed, clipped and aligned using the known reference genome (hg19). The data processed in this way was used for the following analyses.

### Results:

A total of 2446 SNPs were found in the 12 analyzed genes. Significant associations between SNPs and the lipid parameters were found in 4 SNPs. Each of these 4 SNPs was found to be associated and correlated to changes in the lipid and FA levels caused by MPL supplementation, including changes in total cholesterol, LDL, HDL, LPC, as well as in EPA, DHA and AA (in the TG and PL fraction respectively). Associations between SNPs and the lipid parameters could only be evaluated in subjects which finished the clinical trial and which had a known PCa condition (n=114). Since sequencing failed in 2 subjects the study population was n=112.

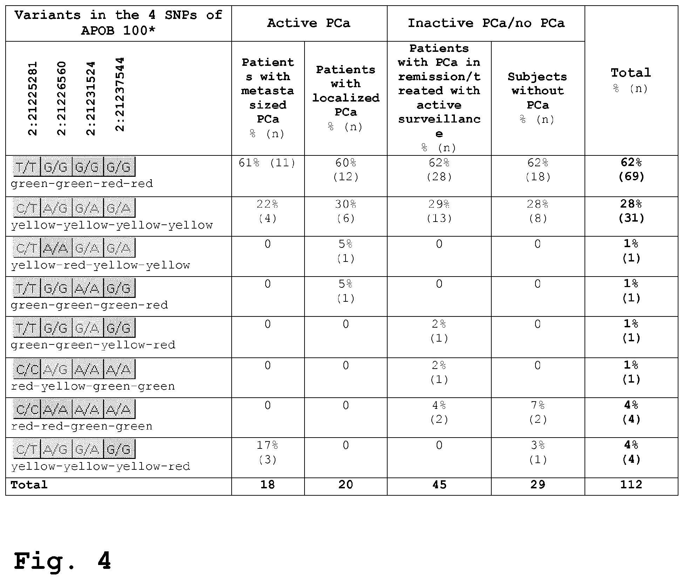

Significant associations between SNPs and the lipid parameters were found for the 4 SNPs indicated in the above Table 1. When combining the allele variations of the 4 SNPs, 8 different allele combinations were found in the present study population (n=112). Figure 4 summarizes the allele combinations and their incidence in the different groups of subjects.

As can be seen in the table depicted in Figure 4, the variation T/T-G/G-G/G-G/G was the most abundant one, found in 69 subjects (62%) and distributed equally in all groups of subjects.

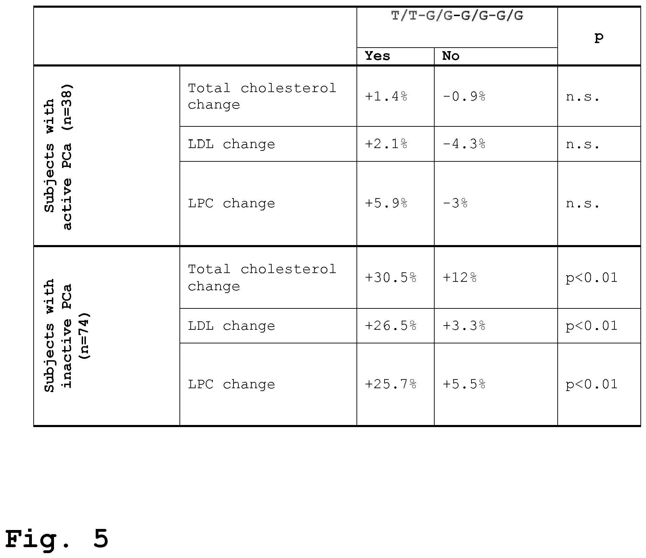

The median relative change of total cholesterol, LDL and LPC after 3 months of MPL supplementation was analyzed in subjects stratified according to the presence of the T/T-G/G-G/G-G/G allele variation. The results are shown in Figure 5. The table in Figure 5 shows a statistically significant difference between subjects with and without T/T-G/G-G/G-G/G in the group of patients without PCa and with inactive PCa, which again demonstrates the effect of the T/T-G/G-G/G-G/G allele variation. In patients suffering from active PCa, which have an increased demand on LPC and LDL, the differences are not significant.

### Literature

[1] Augustsson et al. (2003), "A Prospective Study of Intake of Fish and Marine Fatty Acids and Prostate Cancer." Cancer Epidemiol Biomarkers Prev 12 (1): 64-7.
[2] Szymanski et al. (2010), "Fish Consumption and Prostate Cancer Risk: a Review and Meta-analysis." Am J Clin Nutr 92 (5): 1223-33.
[3] Brown et al. (2006), "Promotion of prostatic metastatic migration towards human bone marrow stoma by Omega 6 and its inhibition by Omega 3 PUFAs." British Journal of Cancer 94 (6): 842-853.
[4] Murakami et al. (2011), "Lipid Mediators in Life Science." Experimental Animals/Japanese Association for Laboratory Animal Science 60 (1): 7-20.
[5] Larsson at al. (2004), "Dietary Long-chain N-3 Fatty Acids for the Prevention of Cancer: a Review of Potential Mechanisms." Am J Clin Nutr 79 (6): 935-45.
[6] Jantscheff et al. (2011) "Lysophosphatidylcholine Pretreatment Reduces VLA-4 and P-Selectin-mediated B16.f10 Melanoma Cell Adhesion in Vitro and Inhibits Metastasis-like Lung Invasion in Vivo." Molecular Cancer Therapeutics 10(1): 186-197.
[7] Zhao et al. (2007) "Plasma Lysophosphatidylcholine Levels: Potential Biomarkers for Colorectal Cancer." Journal of Clinical Oncology: Official Journal of the American Society of Clinical Oncology 25 (19): 2696-2701.
[8] Sambrook, et al. (1989) "Molecular Cloning: A Laboratory Manual" CSH Press, 2nd edition (1989).
[9] Ausubel et al. (1994) "Current Protocols in Molecular Biology" Current Protocols Publishing, New York.
[10] Olivier et al. (2005), "The Invader assay for SNP genotyping." Mutat Res., 573(1-2), 103-10.
[11] Howell et al. (1999), "Dynamic allele-specific hybridization. A new method for scoring single nucleotide polymorphisms." Nat Biotechnol., 17(1), 87-8.
[12] Murphy et al. (2011), "Supplementation with fish oil increases first-line chemotherapy efficacy in patients with advanced non-small cell lung cancer." Cancer, 117(16):3774-80.
[13] Küllenberg et al. (2012), "Health effects of dietary phospholipids." Lipids Health Dis., 11:3.
[14] Fenton et al. (2013), "Immunomodulation by dietary long chain omega-3 fatty acids and the potential for adverse health outcomes." Prostaglandins Leukot Essent Fatty Acids, 89(6):379-90.
[15] Lee & Park (2013), "The association between n-3 polyunsaturated fatty acid levels in erythrocytes and the risk of rheumatoid arthritis in Korean women." Ann Nutr Metab., 63(1-2):88-95.
[16] Bosco et al. (2013), "Effects of increase in fish oil intake on intestinal eicosanoids and inflammation in a mouse model of colitis.", Lipids Health Dis. 12(1):81.
[17] Shinto et al. (2014), "A randomized placebo-controlled pilot trial of omega-3 fatty acids and alpha lipoic acid in Alzheimer's disease." J Alzheimers Dis., 38(1):111-20.
[18] Giles et al. (2013), "Omega-3 fatty acids influence mood in healthy and depressed individuals.", Nutr Rev. 71(11):727-41.
[19] Gow et al. (2013), "Omega-3 fatty acids are inversely related to callous and unemotional traits in adolescent boys with attention deficit hyperactivity disorder." Prostaglandins Leukot Essent Fatty Acids, 88(6):411-8.
[20] Sohrabi et al. (2013) "Evaluation of the effect of omega-3 fatty acids in the treatment of premenstrual syndrome: a pilot trial." Complement Ther Med. 21(3):141-6.
[21] Rossmeisl et al. (2013) "Omega-3 phospholipids from fish suppress hepatic steatosis by integrated inhibition of biosynthetic pathways in dietary obese mice.", Biochim Biophys Acta. 1841(2):267-278.
[22] Tousoulis et al. (2014) "Omega-3 PUFAs improved endothelial function and arterial stiffness with a parallel antiinflammatory effect in adults with metabolic syndrome." J Atheroscleorsis 232(1): 10-6.
[23] Sarbolouki et al. (2013) "Eicosapentaenoic acid improves insulin sensitivity and blood sugar in overweight type 2 diabetes mellitus patients: a double-blind randomised clinical trial." Singapore Med J. 54(7):387-90.
[24] Wen et al. (2013), "Effects of Omega-3 fatty acid on major cardiovascular events and mortality in patients with coronary heart disease: A meta-analysis of randomized controlled trials.", Nutr Metab Cardiovasc Dis., Dec 25. pii: S0939-4753(13)00308-6.
[25] EP2085089
[26] Shirouchi et al. (2007), " Effect of dietary omega 3 phosphatidylcholine on obesity-related disorders in obese Otsuka Long-Evans Tokushima fatty rats." J Agric Food Chem. 55(17):7170-6.

## Claims

1. In vitro method for identifying an individual who benefits from a high omega-3-fatty acid (n-3-FA) diet when being afflicted with prostate cancer, said method comprising
(a) providing a nucleic acid sample of said individual,
(b) detecting the presence of at least one of the following single nucleotide polymorphisms (SNPs): G/G in position 101 of SEQ ID NO:1, G/G in position 101 of SEQ ID NO:2, G/G in position 101 of SEQ ID NO:3, or T/T in position 101 of SEQ ID NO:4, or at least one of the complements thereof, at the corresponding SNP position(s) in the nucleic acid sample,
wherein the presence of at least one of the SNPs at the corresponding SNP position(s) indicates that said individual benefits from a high n-3-FA diet when being afflicted with prostate cancer.

2. In vitro method for identifying an individual who has a reduced risk of developing an aggressive prostate cancer type and/or metastasis when being afflicted with a prostate cancer disease and subjected to a high omega-3-fatty acid (n-3-FA) diet, said method comprising
(a) providing a nucleic acid sample of said individual,
(b) detecting the presence of at least one of following single nucleotide polymorphisms (SNPs): G/G in position 101 of SEQ ID NO:1, G/G in position 101 of SEQ ID NO:2, G/G in position 101 of SEQ ID NO:3, or T/T in position 101 of SEQ ID NO:4, or at least one of the complements thereof, at the corresponding SNP position(s) in the nucleic acid sample,
wherein the presence of at least one of the SNPs at the corresponding SNP position(s) indicates that said individual has a reduced risk of developing an aggressive cancer type and/or metastasis.

3. In vitro method of assigning a patient to a diet suitable for reducing the severity of prostate cancer, said method comprising
(a) providing a nucleic acid sample of a patient who has been diagnosed with prostate cancer;
(b) detecting the presence of at least one of the following single nucleotide polymorphisms (SNPs): G/G in position 101 of SEQ ID NO:1, G/G in position 101 of SEQ ID NO:2, G/G in position 101 of SEQ ID NO:3, or T/T in position 101 of SEQ ID NO:4, or at least one of the complements thereof, at the corresponding SNP position(s) in the nucleic acid sample,
(c) assigning said patient to a high omega-3-fatty acid (n-3-FA) diet if at least one of the SNPs at the corresponding SNP position(s) is detected in step (b).

4. In vitro method of determining the responsiveness of prostate cancer to a high omega-3-fatty acid (n-3-FA) diet, said method comprising
(a) providing a nucleic acid sample of a patient who has been diagnosed with prostate cancer;
(b) detecting the presence of at least one of the following single nucleotide polymorphisms (SNPs): G/G in position 101 of SEQ ID NO:1, G/G in position 101 of SEQ ID NO:2, G/G in position 101 of SEQ ID NO:3, or T/T in position 101 of SEQ ID NO:4,, or at least one of the complements thereof, at the corresponding SNP position(s) in the nucleic acid sample,
(c) classifying said prostate cancer as responsive to a high omega-3-fatty acid (n-3-FA) diet if at least one of the SNPs at the corresponding SNP position(s) is detected in step (b).

5. In vitro method for identifying an individual who is able to increase the plasma levels of lysophosphatidylcholine (LPC) and/or low-density lipoprotein (LDL) and/or total cholesterol in response to omega-3-fatty acid (n-3-FA) consumption, said method comprising
(a) providing a nucleic acid sample of said individual;
(b) detecting the presence of at least one of the following single nucleotide polymorphisms (SNPs): G/G in position 101 of SEQ ID NO:1, G/G in position 101 of SEQ ID NO:2, G/G in position 101 of SEQ ID NO:3, or T/T in position 101 of SEQ ID NO:4, or at least one of the complements thereof, at the corresponding SNP position(s) in the nucleic acid sample,
wherein the presence of at least one of the SNPs at the corresponding SNP position(s) indicates that said individual is able to increase the plasma levels of lysophosphatidylcholine (LPC) and/or low-density lipoprotein (LDL) and/or total cholesterol in response to omega-3-fatty acid (n-3-FA) consumption.

6. Method according to any of claims 1 to 5, wherein detecting in step (b) involves a PCR, an RT-PCR, an oligonucleotide ligation assay, a microarray and/or sequencing.

7. Method according to any of claims 1 to claim 6, wherein said individual or patient is a biallelic individual, preferably a human.

8. Method according to any of claims 1 to 7, wherein two, three or four of the SNPs shown in position 101 of SEQ ID NO:1-4, or the complements thereof, are detected in parallel at the corresponding SNP position(s) in step (b).

9. Method according to any of claims 1 to 8, wherein said omega-3-fatty acid (n-3-FA) diet comprises fish, fish oil, n-3 FA methyl esters, free n-3 FA and/or marine phospholipids.

10. Method according to claim 9, wherein said diet provides for a weekly n-3 FA intake of between 0.7 and 35 g, and preferably for a daily intake of between 0.4 and 0.5 g.

11. Method according to any of claims 1 to 10, wherein said nucleic acid sample is derived from a blood sample or a mucosal swab of the individual to be tested.

12. Method according to any of claims 1 to 11, wherein said nucleic acid sample comprises DNA or RNA of the individual or patient to be tested.

## Patentansprüche

1. In vitro-Verfahren zur Identifizierung eines Individuums, das bei Erkrankung an Prostatakrebs von einer Ernährung mit einer hohen Menge an Omega-3-Fettsäuren (n-3-FA) profitieren würde, bei dem man
(a) eine Nukleinsäure-Probe von diesem Individuum bereitstellt,
(b) das Vorhandensein von mindestens einem der folgenden Einzelnukleotid-Polymorphismen (SNPs): G/G in Position 101 von SEQ ID NO:1, G/G in Position 101 von SEQ ID NO:2, G/G in Position 101 von SEQ ID NO:3 oder T/T in Position 101 von SEQ ID NO:4, oder von mindestens einer dazu komplementären Sequenz an der/den entsprechenden SNP-Position(en) in der Nukleinsäure-Probe nachweist,
wobei das Vorhandensein von mindestens einem der SNPs an der/den entsprechenden SNP-Position(en) darauf verweist, dass das Individuum bei Erkrankung an Prostatakrebs von einer Ernährung mit einer hohen Menge an Omega-3-Fettsäuren (n-3-FA) profitiert.

2. In vitro-Verfahren zur Identifizierung eines Individuums, das bei Erkrankung an Prostatakrebs ein verringertes Risiko aufweist, eine aggressive Art von Prostatakrebs und/oder Metastasen zu entwickeln, wenn es einer Ernährung mit einer hohen Menge an Omega-3-Fettsäuren (n-3-FA) unterzogen wird, bei dem man
(a) eine Nukleinsäure-Probe von diesem Individuum bereitstellt,
(b) das Vorhandensein von mindestens einem der folgenden Einzelnukleotid-Polymorphismen (SNPs): G/G in Position 101 von SEQ ID NO:1, G/G in Position 101 von SEQ ID NO:2, G/G in Position 101 von SEQ ID NO:3 oder T/T in Position 101 von SEQ ID NO:4, oder von mindestens einer dazu komplementären Sequenz an der/den entsprechenden SNP-Position(en) in der Nukleinsäure-Probe nachweist,
wobei das Vorhandensein von mindestens einem der SNPs an der/den entsprechenden SNP-Position(en) darauf verweist, dass das Individuum ein verringertes Risiko aufweist, eine aggressive Art von Prostatakrebs und/oder Metastasen zu entwickeln.

3. In vitro-Verfahren für die Zuordnung eines Patienten an eine Ernährung, die geeignet ist, die Ernsthaftigkeit von Prostatakrebs zu verringern, bei dem man
(a) eine Nukleinsäure-Probe eines Patienten bereitstellt, bei dem Prostatakrebs diagnostiziert wurde;
(b) das Vorhandensein von mindestens einem der folgenden Einzelnukleotid-Polymorphismen (SNPs): G/G in Position 101 von SEQ ID NO:1, G/G in Position 101 von SEQ ID NO:2, G/G in Position 101 von SEQ ID NO:3 oder T/T in Position 101 von SEQ ID NO:4, oder von mindestens einer dazu komplementären Sequenz an der/den entsprechenden SNP-Position(en) in der Nukleinsäure-Probe nachweist,
(c) dem Patienten eine Ernährung mit einer hohen Menge an Omega-3-Fettsäuren (n-3-FA) zuordnet, wenn in Schritt (b) mindestens einer der SNPs an der/den entsprechenden SNP-Position(en) nachgewiesen wird.

4. In vitro-Verfahren zur Bestimmung der Ansprechbarkeit von Prostatakrebs auf eine Ernährung mit einer hohen Menge an Omega-3-Fettsäuren (n-3-FA), bei dem man
(a) eine Nukleinsäure-Probe eines Patienten bereitstellt, bei dem Prostatakrebs diagnostiziert wurde;
(b) das Vorhandensein von mindestens einem der folgenden Einzelnukleotid-Polymorphismen (SNPs): G/G in Position 101 von SEQ ID NO:1, G/G in Position 101 von SEQ ID NO:2, G/G in Position 101 von SEQ ID NO:3 oder T/T in Position 101 von SEQ ID NO:4, oder von mindestens einer dazu komplementären Sequenz an der/den entsprechenden SNP-Position(en) in der Nukleinsäure-Probe nachweist,
(c) den Prostatakrebs als einen solchen einstuft, der auf eine Ernährung mit einer hohen Menge an Omega-3-Fettsäuren (n-3-FA) anspricht, wenn in Schritt (b) mindestens einer der SNPs an der/den entsprechenden SNP-Position(en) nachgewiesen wird.

5. In vitro-Verfahren zur Identifizierung eines Individuums, das in der Lage ist, die Plasmamengen an Lysophosphatidylcholin (LPC) und/oder Low-Density-Lipoprotein (LDL) und/oder Gesamt-Cholesterol in Reaktion auf den Verzehr von Omega-3-Fettsäuren (n-3-FA) zu erhöhen, bei dem man
(a) eine Nukleinsäure-Probe von diesem Individuum bereitstellt,
(b) das Vorhandensein von mindestens einem der folgenden Einzelnukleotid-Polymorphismen (SNPs): G/G in Position 101 von SEQ ID NO:1, G/G in Position 101 von SEQ ID NO:2, G/G in Position 101 von SEQ ID NO:3 oder T/T in Position 101 von SEQ ID NO:4, oder von mindestens einer dazu komplementären Sequenz an der/den entsprechenden SNP-Position(en) in der Nukleinsäure-Probe nachweist,
wobei das Vorhandensein von mindestens einem der SNPs an der/den entsprechenden SNP-Position(en) darauf verweist, dass das Individuum in der Lage ist, die Plasmamengen an Lysophosphatidylcholin (LPC) und/oder Low-Density-Lipoprotein (LDL) und/oder Gesamt-Cholesterol in Reaktion auf den Verzehr von Omega-3-Fettsäuren (n-3-FA) zu erhöhen

6. Verfahren nach einem der Ansprüche 1-5, bei dem der Nachweis in Schritt (b) eine PCR, eine RT-PCR, einen Oligonukleotid-Ligations-Assay, einen Microarray und oder eine Sequenzierung umfasst.

7. Verfahren nach einem der Ansprüche 1-6, bei dem das Individuum oder der Patient ein biallelisches Individuum ist, vorzugsweise ein Mensch.

8. Verfahren nach einem der Ansprüche 1-7, bei dem in Schritt (b) zwei, drei oder vier der in Position 101 von SEQ ID NOs:1-4 gezeigten SNPs oder die dazu komplementären Sequenzen parallel an der/den entsprechenden SNP-Position(en) nachgewiesen werden.

9. Verfahren nach einem der Ansprüche 1-8, bei dem die Ernährung mit einer hohen Menge an Omega-3-Fettsäuren (n-3-FA) Fisch, Fischöl, n-3-FA-Methylester, freie n-3-FA und/oder marine Phospholipide umfasst.

10. Verfahren nach Anspruch 9, bei dem die Ernährung für eine wöchentliche Aufnahme von n-3 FA zwischen 0,7 und 35 g sorgt, und vorzugsweise für eine tägliche Aufnahme zwischen 0,4 und 0,5 g.

11. Verfahren nach einem der Ansprüche 1-10, bei dem die Nukleinsäure-Probe von einer Blutprobe oder von einem Schleimhautabstrich des zu untersuchenden Individuums stammt.

12. Verfahren nach einem der Ansprüche 1-11, bei dem die Nukleinsäure-Probe DNA oder RNA des zu untersuchenden Individuums umfasst.

## Revendications

1. Procédé in vitro pour identifier un individu qui tire bénéfice d'un régime à forte teneur en acides gras oméga-3 (AG n-3) tout en étant atteint d'un cancer de la prostate, ledit procédé comprenant les étapes consistant à
(a) fournir un échantillon d'acide nucléique dudit individu,
(b) détecter la présence d'au moins l'un des polymorphismes d'un seul nucléotide (SNP) suivants : G/G en position 101 de la Séquence N° 1, G/G en position 101 de la Séquence N° 2, G/G en position 101 de la Séquence N° 3, ou T/T en position 101 de la Séquence N° 4, ou d'au moins l'un de leurs compléments, à la (aux) position(s) de SNP individuel(s) correspondante(s) dans l'échantillon d'acide nucléique,
la présence d'au moins l'un des SNP à la (aux) position(s) de SNP correspondante(s) indiquant que ledit individu tire bénéfice d'un régime à forte teneur en AG n-3 tout en étant atteint d'un cancer de la prostate.

2. Procédé in vitro pour identifier un individu qui a un risque réduit de développer un type agressif de cancer de la prostate et/ou une dissémination de métastases lorsqu'il est atteint d'une maladie cancéreuse de la prostate et soumis à un régime à forte teneur en acides gras oméga-3 (AG n-3), ledit procédé comprenant les étapes consistant à
(a) fournir un échantillon d'acide nucléique dudit individu,
(b) détecter la présence d'au moins l'un des polymorphismes d'un seul nucléotide (SNP) suivants : G/G en position 101 de la Séquence N° 1, G/G en position 101 de la Séquence N° 2, G/G en position 101 de la Séquence N° 3, ou T/T en position 101 de la Séquence N° 4, ou d'au moins l'un de leurs compléments, à la (aux) position(s) de SNP correspondante(s) dans l'échantillon d'acide nucléique,
la présence qu'au moins l'un des SNP à la (aux) position(s) de SNP correspondante(s) indiquant que ledit individu a un risque réduit de développer un type agressif de cancer et/ou une dissémination de métastases.

3. Procédé in vitro d'affectation d'un patient à un régime approprié à réduire la gravité d'un cancer de la prostate, ledit procédé comprenant les étapes consistant à
(a) fournir un échantillon d'acide nucléique d'un patient chez lequel un cancer de la prostate a été diagnostiqué,
(b) détecter la présence d'au moins l'un des polymorphismes d'un seul nucléotide (SNP) suivants : G/G en position 101 de la Séquence N° 1, G/G en position 101 de la Séquence N° 2, G/G en position 101 de la Séquence N° 3, ou T/T en position 101 de la Séquence N° 4, ou d'au moins l'un de leurs compléments, à la (aux) position(s) de SNP correspondante(s) dans l'échantillon d'acide nucléique,
(c) affecter ledit patient à un régime à forte teneur en acides gras oméga-3 (AG n-3) si au moins l'un des SNP à la (aux) position(s) de SNP correspondante(s) est détecté dans l'étape (b).

4. Procédé in vitro de détermination de l'aptitude d'un cancer de la prostate à répondre à un régime à forte teneur en acides gras oméga-3 (AG n-3), ledit procédé comprenant les étapes consistant à
(a) fournir un échantillon d'acide nucléique d'un patient chez lequel un cancer de la prostate a été diagnostiqué,
(b) détecter la présence d'au moins l'un des polymorphismes d'un seul nucléotide (SNP) suivants : G/G en position 101 de la Séquence N° 1, G/G en position 101 de la Séquence N° 2, G/G en position 101 de la Séquence N° 3, ou T/T en position 101 de la Séquence N° 4, ou d'au moins l'un de leurs compléments, à la (aux) position(s) de SNP correspondante(s) dans l'échantillon d'acide nucléique,
(c) classer ledit cancer de la prostate comme répondant à un régime à forte teneur en acides gras oméga-3 (AG n-3) si au moins l'un des SNP à la (aux) position(s) de SNP correspondante(s) est détecté dans l'étape (b).

5. Procédé in vitro pour identifier un individu qui est apte à présenter une augmentation des taux plasmatiques de lysophosphatidylcholine (LPC) et/ou de cholestérol des lipoprotéines de basse densité (LDL) et/ou de cholestérol total en réponse à une consommation d'acides gras oméga-3 (AG n-3), ledit procédé comprenant les étapes consistant à
(a) fournir un échantillon d'acide nucléique dudit individu ;
(b) détecter la présence d'au moins l'un des polymorphismes d'un seul nucléotide (SNP) suivants : G/G en position 101 de la Séquence N° 1, G/G en position 101 de la Séquence N° 2, G/G en position 101 de la Séquence N° 3, ou T/T en position 101 de la Séquence N° 4, ou d'au moins l'un de leurs compléments, à la (aux) position(s) de SNP correspondante(s) dans l'échantillon d'acide nucléique,
la présence d'au moins l'un des SNP à la (aux) position(s) de SNP correspondante(s) indiquant que ledit individu est apte à présenter une augmentation des taux plasmatiques de lysophosphatidylcholine (LPC) et/ou de cholestérol des lipoprotéines de basse densité (LDL) et/ou de cholestérol total en réponse à une consommation d'acides gras oméga-3 (AG n-3)

6. Procédé selon l'une quelconque des revendications 1 à 5, dans lequel la détection dans l'étape (b) fait intervenir une PCR, une RT-PCR, un essai de ligature d'oligonucléotides, un microréseau et/ou un séquençage.

7. Procédé selon l'une quelconque des revendications 1 à 6, dans lequel ledit individu ou patient est un individu biallélique, de préférence un humain.

8. Procédé selon l'une quelconque des revendications 1 à 7, dans lequel deux, trois ou quatre des SNP présentés en position 101 des Séquences N° 1 - 4, ou leurs compléments, sont détectés en parallèle à la (aux) position(s) de SNP correspondante(s) dans l'étape (b).

9. Procédé selon l'une quelconque des revendications 1 à 8, dans lequel ledit régime riche en acides gras oméga 3 (AG n-3) comprend le poisson, l'huile de poisson, des esters méthyliques d'AG n-3, des AG n-3 libres et/ou des phospholipides marins.

10. Procédé selon la revendication 9, dans lequel ledit régime procure un apport hebdomadaire d'AG n-3 compris entre 0,7 et 35 g, et de préférence un apport journalier compris entre 0,4 et 0,5 g.

11. Procédé selon l'une quelconque des revendications 1 à 10, dans lequel ledit échantillon d'acide nucléique est issu d'un échantillon de sang ou d'un prélèvement muqueux de l'individu à tester.

12. Procédé selon l'une quelconque des revendications 1 à 11, dans lequel ledit échantillon d'acide nucléique comprend de l'ADN ou de l'ARN de l'individu ou du patient à tester.
